# EUROPÄISCHE PATENTSCHRIFT

(11) **EP 1 506 170 B1**
(45) Veröffentlichungstag und Bekanntmachung des Hinweises auf die Patenterteilung: **27.02.2008**
(21) Anmeldenummer: 03730023.3
(22) Anmeldetag: 13.05.2003
(51) Int. Cl.: C07C 315/00, C07C 317/46

(54) **VERFAHREN ZUR HERSTELLUNG VON BICALUTAMID**
METHOD FOR PRODUCING BICALUTAMIDE
PROCEDE DE PREPARATION DE BICALUTAMIDE

(30) Priorität: 17.05.2002 DE 10222104
(43) Veröffentlichungstag der Anmeldung: 16.02.2005
(73) Patentinhaber: Helm AG, 20097 Hamburg (DE); CF Pharma Gyogyszergyarto Kft., 1097 Budapest (HU)
(72) Erfinder: BOR, Adám, H-2040 Budaörs (HU); OROSZ, György, H-1143 Budapest (HU); LUKACS, Ferenc, H-2143 Kistarcsa (HU); SCHNEIDER, Géza, H-1097 Budapest (HU)
(74) Vertreter: Teipel, Stephan
(86) Internationale Anmeldenummer: PCT/EP2003/004999
(87) Internationale Veröffentlichungsnummer: WO 2003/097590

(56) Entgegenhaltungen:
- WO-A-01/00608
- WO-A-01/28990
- DE-A- 3 304 054
- US-A1- 2003 073 742
- CULVENOR, C.C.J. ET AL.: J. CHEM. SOC., 1949, Seiten 2198-2206, XP001091304
- KLAMANN, D. (ED.): "Methoden der organischen Chemie (Houben-Weyl), Band E11 Organische Schwefel-Verbindungen" 1985 , GEORG THIEME VERLAG , STUTTGART XP002253733 Seite 1145 -Seite 1150

## Beschreibung

Die vorliegende Erfindung betrifft ein Verfahren zur Herstellung von (±)-N-(4'-Cyano-3'-trifluormethyl)-3-(4"-fluorphenylsulfonyl)-2-hydroxy-2-methyl-propionamid sowie seiner R-(-)- und S-(+)-Enantiomeren.

N-(4'-Cyano-3'-trifluormethyl)-3-(4"-fluorphenylsulfonyl)-2-hydroxy-2-methyl-propionamid ist auch unter dem INN-Namen Bicalutamid bekannt und gehört zur Klasse der Acylanilide. Bicalutamid hat die folgende chemische Strukturformel:

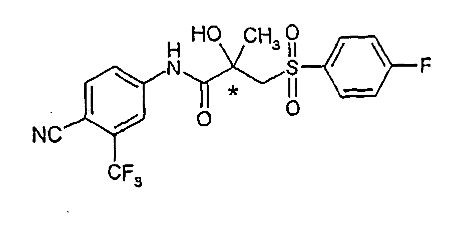

Die Verbindung besitzt ein asymmetrisches C-Atom (*) und kann daher sowohl in seiner racemischen Form als (±)-Bicalutamid als auch als R-(-)- bzw. S-(+)-Enantiomeres auftreten.

Zahlreiche Acylanilide besitzen antiandrogene Eigenschaften und werden heute als nichtsteroidale, periphere Antiandrogene bezeichnet. Diese Verbindungen sind zur Therapie von androgen-bedingten Krankheiten, wie z.B. gutartigen oder bösartigen Erkrankungen der Prostata, Hirsutismus, Akne etc. geeignet.

Bicalutamid wird in Form seines Racemates unter dem Handelsnamen Casodex^{®} seit 1995 als Mittel gegen Prostatakarzinome vertrieben.

Acylanilide mit antiandrogenen Eigenschaften, die auch Bicalutamid umfassen, werden beispielsweise in dem EP 0 100 172 offenbart. Dieses Dokument beschreibt auch verschiedene Synthesewege, die sich auf Bicalutamid gelesen wie folgt zusammenfassen lassen:

In einem ersten Syntheseweg wird ausgehend von Methylmethacrylat zunächst ein Epoxid hergestellt, daß mit p-Fluorthiophenol unter Addition geöffnet wird. Nach einer Esterspaltung wird das entsprechende Amid gebildet und im letzten Schritt wird das Sulfid mittels einer Persäure wie m-Chlorperbenzoesäure (m-CPBA) zum Sulfon oxidiert. Dieser Syntheseweg wird durch das nachfolgende Reaktionsschema veranschaulicht:

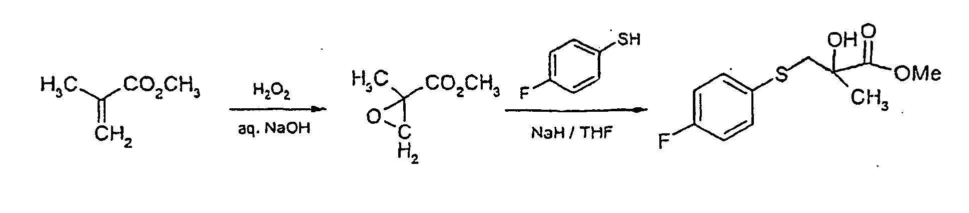

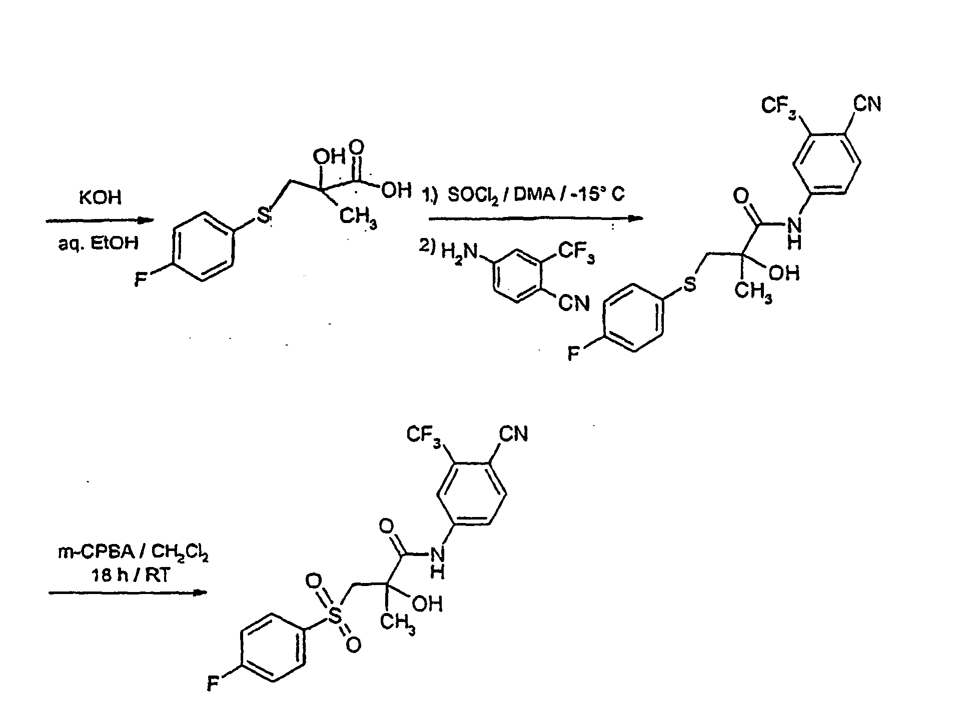

In einem zweiten Reaktionsweg wird ausgehend von Bromaceton zunächst mit p-Fluorthiophenol das entsprechende Sulfid gebildet, in das dann über eine Cyanhydrin-Reaktion die Säure eingeführt wird. Nach der Amidbildung mit 3-Trifluormethyl-4-cyanoanilin wird dann im letzten Schritt wieder das Sulfid zum Sulfon oxidiert. Dieser Syntheseweg wird durch das nachfolgende Reaktionsschema veranschaulicht:

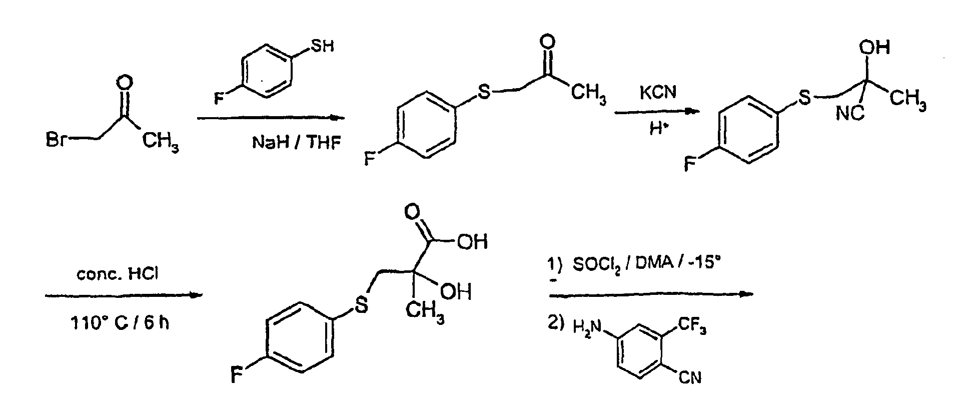

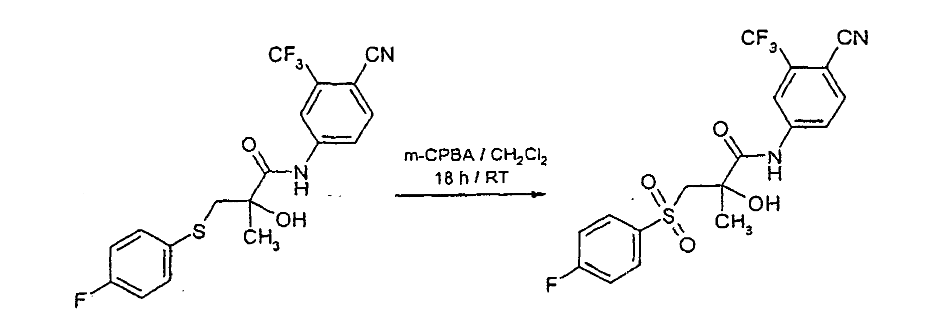

In einem weiteren in dem EP 0 100 172 offenbarten Syntheseweg wird zunächst aus Methacryloylchlorid mit 3-Trifluormethyl-4-cyanoanilin das entsprechende Säureamid gebildet, das dann zu einem Oxiran umgesetzt wird. Dieses wird mit p-Fluorthiophenol unter Addition geöffnet und schließlich wird wieder das Sulfid zum Sulfon oxidiert. Dieser Reaktionsweg wird durch das nachfolgende Reaktionsschema veranschaulicht:

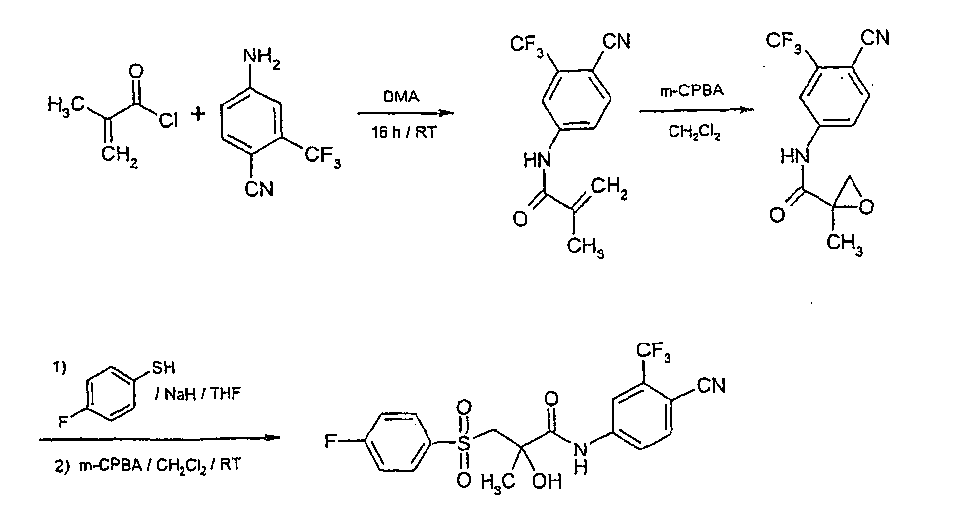

Ein Syntheseweg für die Herstellung der beiden möglichen Enantiomeren des Bicalutamids ist in der WO 98/55153 offenbart. Diese Synthese geht von R-Prolin bzw. S-Prolin aus. Hieraus wird zunächst durch eine optisch induzierte Bromlactonisierung das entsprechend substituierte Propionsäureenantiomere gewonnen. Im weiteren Verlauf wird zuerst mit 3-Trifluormethyl-4-cyanoanilin das entsprechende Amid gebildet, bevor wiederum p-Fluorthiophenol unter stark basischen Bedingungen zum Aufbau des vollständigen Molekülgerüsts eingesetzt wird. Abschließend wird wieder das Sulfid zum entsprechenden Sulfon oxidiert. Dieser Reaktionsweg wird auf R-(-)-Bicalutamid gelesen durch das folgende Reaktionsschema verdeutlicht:

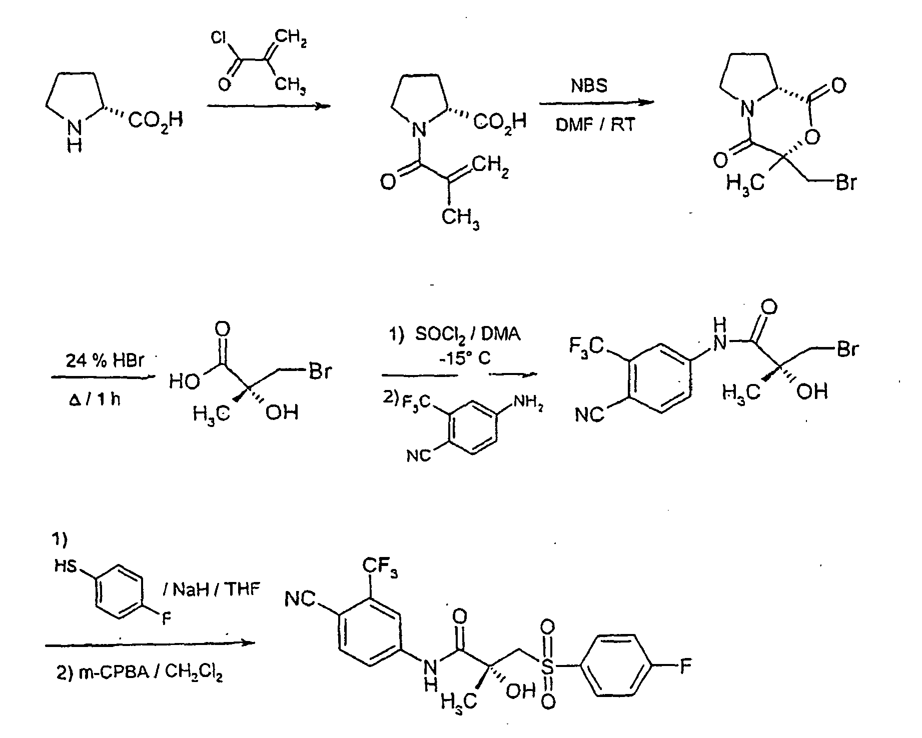

Ein weiteres alternatives Verfahren zur Herstellung von Bicalutamid wird in der WO 01/00608 beschrieben. Dieses Verfahren geht von 2,3-Dihydroxy-2-methylpropionsäure aus, und setzt im Laufe des Verfahrens ebenfalls mit 3-Trifluormethyl-4-cyanoanilin zum entsprechenden Amid und schließlich mit p-Fluorthiophenol zum entsprechenden Sulfid um. Dieses wird anschließend zum gewünschten Sulfon oxidiert. Diese Synthese wird durch das nachfolgende Reaktionsschema veranschaulicht:

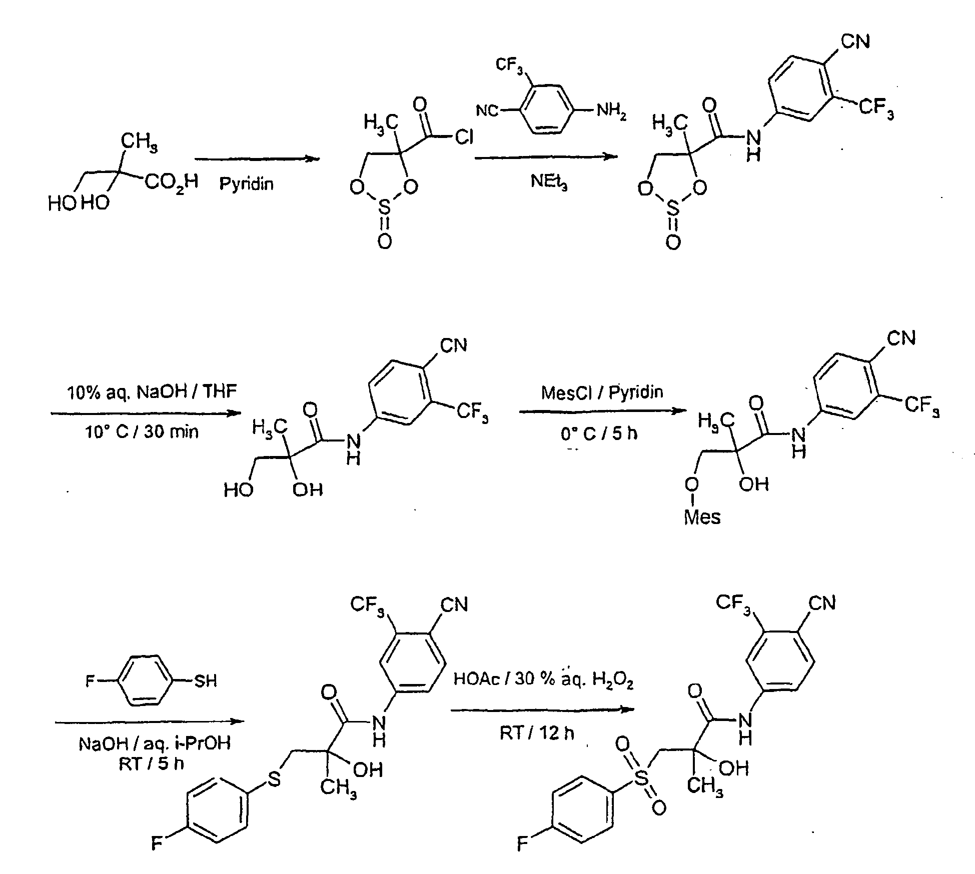

Schließlich offenbart die WO 01/28990 ein Verfahren zur asymmetrischen Synthese eines Enantiomers eines Acylanilids unter Öffnung einer Ringsstruktur. In den Beispielen wird Bicalutamid durch Reaktion mit p-Fluorthiophenol und anschließender Oxidation des erhaltenen Sulfids zum entsprechenden Sulfon mittels m-Chlorperbenzoesäure beschrieben.

Alle vorstehend beschriebenen Verfahren aus dem Stand der Technik verwenden zur Herstellung von Bicalutamid prinzipiell folgende Reaktionsschritte: 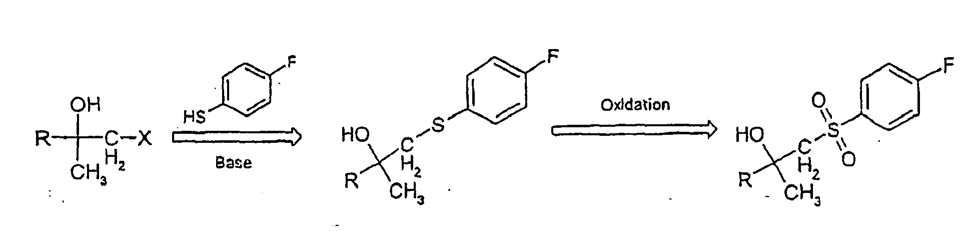 wobei R der jeweilige Restteil des Bicalutamid-Gerüsts oder einer entsprechenden Vorstufe ist und X entweder eine übliche Abgangsgruppe ist, wie z.B. Cl, Br, I, MesO, TosO, etc. oder zusammen mit dem a-ständigen C-Atom ein entsprechendes Epoxid bildet, das dann unter basischen Bedingungen p-Fluorthiophenol zu einem Sulfid addiert, das im nächsten Schritt zum entsprechenden Sulfon oxidiert wird. Wie aus den gezeigten Beispielen hervorgeht, muß diese Reaktionsfolge nicht am Ende einer Bicalutamid-Synthese auftreten, sondern kann auch in der Mitte der Reaktionsfolge verwendet werden. Ebenso muß die Oxidation nicht zwangsläufig unmittelbar der Bildung des Sulfids folgen, sondern kann auch später durchgeführt werden. Beide Einzelschritte werden aber in allen Bicalutamid-Synthesen verwendet.

Die Verwendung von p-Fluorthiophenol und die daraus folgende notwendige Oxidation des gebildeten Sulfids zum gewünschten Sulfon weist jedoch zahlreiche Nachteile auf. Erstens handelt es sich bei p-Fluorthiophenol um ein toxisches und stark reizendes Material, das entsprechende Vorkehrungen notwendig macht, um diese Verbindung im technischen Maßstab nutzen zu können. Zweitens ist p-Fluorthiophenol nur schwierig in isomerenreiner Form zu erhalten. Eine entsprechende Aufreinigung ist aufwendig. Drittens ist für die Addition des p-Fluorthiophenols eine starke Base notwendig. Die meisten Syntheseprozesse verwenden Natriumhydrid, was jedoch den Einsatz von absoluten Lösungsmitteln notwendig macht und was die Reaktionsführung im technischen Maßstab durch die sehr große Hydrolyseempfindlichkeit erschwert. Die Verwendung schwächerer Basen erfordert den zusätzlichen Schritt der Einführung einer reaktiven Abgangsgruppe. Viertens muß für die Oxidation des Sulfids zum Sulfon ein starkes Oxidationsmittel eingesetzt werden. Im genannten Stand der Technik wurden als Oxidationsmittel m-Chlorperbenzoesäure, Mischungen von H₂O₂ mit organischen Säuren und Oxone^{®} (2 KHSO₅·KHSO₄·K₂SO₄-Salz) beschrieben. m-Chlorperbenzoesäure ist jedoch relativ teuer, Mischungen von H₂O₂ mit organischen Säuren führen zu Persäuren, die im technischen Maßstab gefährlich zu handhaben sind, und die Verwendung von Oxone^{®} erfordert zur Erreichung akzeptabler Ausbeuten die Verwendung von Phasentransfer-Bedingungen, die technisch aufwendig sind.

Es besteht somit weiterhin ein Bedürfnis nach einem wirtschaftlichen und einfach durchzuführenden Verfahren zur Herstellung von Bicalutamid, das vorzugsweise das Bicalutamid entweder in racemischer oder in enantiomerenreiner Form zur Verfügung stellt. Außerdem sollte das Bicalutamid möglichst in der zur Anwendung in Arzneimitteln notwendigen Reinheit erhalten werden.

Eine Aufgabe der vorliegenden Erfindung besteht somit darin, ein Verfahren zur Herstellung von Bicalutamid zur Verfügung zu stellen, das die vorstehend genannten Nachteile überwindet.

Es wurde nun überraschend gefunden, daß Bicalutamid auch ohne die Verwendung von p-Fluorthiophenol und der entsprechend notwendigen Oxidation des entstandenen Sulfids zum Sulfon mittels einer Umsetzung eines Epoxids oder einer Epoxid-Vorstufe mit einem p-Fluorphenylsulfinat-Salz in einfacher Weise erhalten werden kann.

Die vorliegende Erfindung betrifft somit ein Verfahren zur Herstellung von Bicalutamid, worin ein Epoxid der allgemeinen Formel (I) 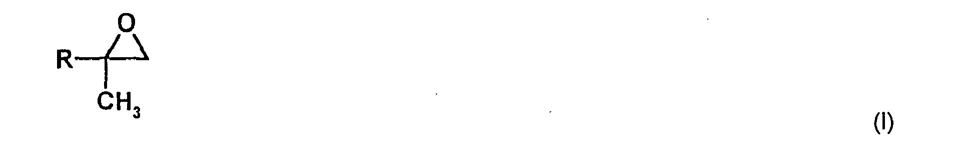 worin R ein Rest der Formel (II) 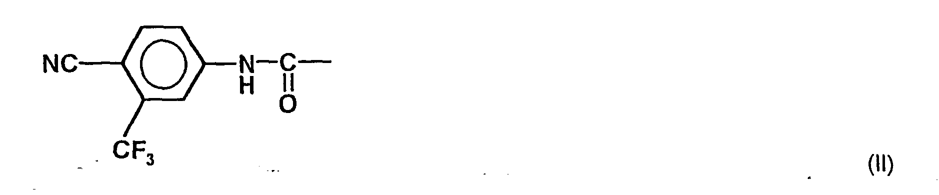 oder eine Vorstufe davon ist, mit einem p-Fluorphenylsulfinat-Salz umgesetzt wird und, wenn R eine Vorstufe des Restes der Formel (II) ist, diese in einen Rest der Formel (II) überführt wird.

Diese Reaktion wird durch das folgende Reaktionsschema anhand einer Umsetzung mit dem bevorzugten Natrium-p-fluorphenylsulfinat veranschaulicht:

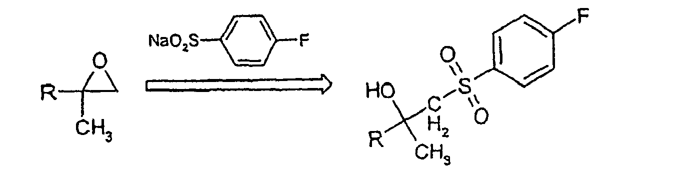

Alternativ kann in dem erfindungsgemäßen Verfahren statt dem Epoxid eine Epoxid-Vorstufe eingesetzt werden, nämlich eine Verbindung der allgemeinen Formel (III) 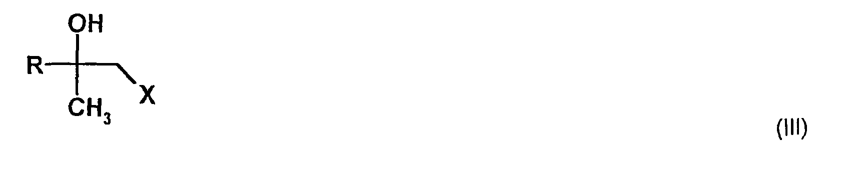 worin R wie oben definiert ist und X eine Abgangsgruppe ist.

Das erfindungsgemäße Verfahren vermeidet somit die Verwendung des toxischen p-Fluorthiophenols und die Notwendigkeit eines Oxidationsschrittes durch die Verwendung eines p-Fluorphenylsulfinats als Epoxid-öffnendes Reagenz. Hierdurch wird direkt das gewünschte Produkt Bicalutamid oder eine Vorstufe davon, die jedoch bereits die gewünschte Sulfongruppe enthält, erhalten.

Das Epoxid der allgemeinen Formel (I) kann entweder als Edukt in dem erfindungsgemäßen Verfahren eingesetzt werden oder durch eine Ringschlußreaktion aus einer Verbindung der allgemeinen Formel (III) 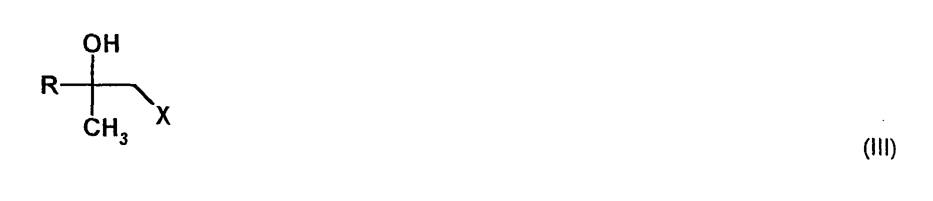 worin R wie vorstehend definiert ist und X eine Abgangsgruppe ist, erhalten werden. Falls gewünscht kann das als Zwischenprodukt erhaltene Epoxid isoliert und gereinigt werden, vorzugsweise wird die Reaktion jedoch ohne Isolierung des Epoxids als Eintopfreaktion fortgeführt. Dies vermindert den Zeit- und Arbeitsaufwand und damit die Synthesekosten und ermöglicht es trotzdem, das gewünschte Bicalutamid in ausreichender Reinheit zu erhalten. Alternativ kann, wie vorstehend bereits ausgeführt, die Verbindung der allgemeinen Formel (III) ohne zwischengeschaltete Oxiranbildung unmittelbar mit dem p-Fluorphenylsulfinat-Salz umgesetzt werden.

In dem erfindungsgemäßen Verfahren wird das eingesetzte Epoxid bzw. dessen offenkettige Vorstufe vorzugsweise so gewählt, daß bei der Umsetzung mit dem p-Fluorphenylsulfinat unmittelbar das gewünschte Bicalutamid erhalten' wird. In diesem Fall steht R in den allgemeinen Formeln (1) und (III) für einen Rest der Formel (II)

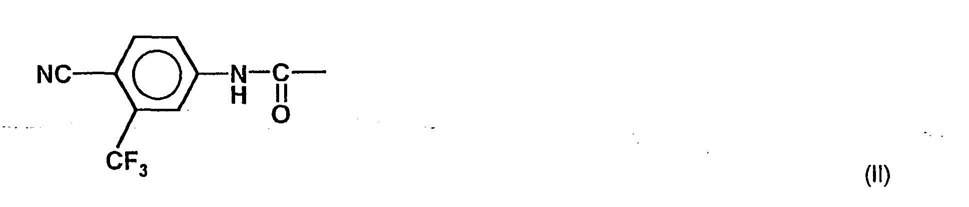

Die erfindungsgemäße Umsetzung des Epoxids mit dem p-Fluorphenylsulfinat-Salz muß jedoch nicht unbedingt am Ende des Synthesewegs zur Herstellung von Bicalutamid stehen. Daher kann R in den allgemeinen Formeln (I) und (III) auch so gewählt werden, daß das in der Reaktion aus dem Epoxid mit dem p-Fluorphenylsulfinat-Salz erhaltene Produkt als Vorstufe für die weitere Synthese von Bicalutamid geeignet ist. Hierzu wird R so gewählt, daß R als Vorstufe für einen Rest der Formel (II) 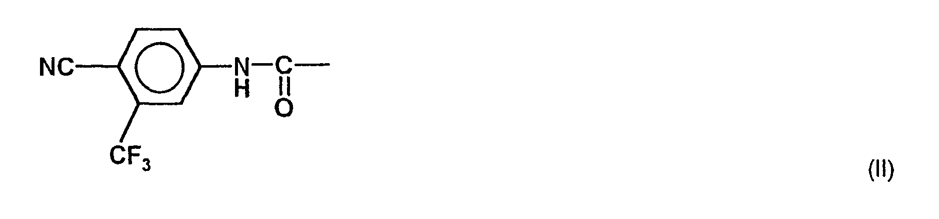 dient. Als Vorstufe für einen Rest der Formel (II) eignet sich beispielsweise der Rest -COY worin Y eine zur nachfolgenden Amidbildung geeignete Gruppe ist. In diesem Fall kann das in der Umsetzung des Epoxids mit dem p-Fluorphenylsulfinat-Salz erhaltene Zwischenprodukt mit 3-Trifluormethyl-4-cyanoanilin leicht zu dem gewünschten Bicalutamid weiter umgesetzt werden. Als Vorstufen für einen Rest der Formel (II) eignen sich insbesondere Carboxyl, Säurehalogenide wie Säurechlorid, normale Ester und aktivierte Ester. Geeignete Reste sind dem Fachmann bekannt und können entsprechend der Reaktionsführung gewählt werden.

Wenn in dem erfindungsgemäßen Verfahren nicht von einem Epoxid der allgemeinen Formel (I), sondern von einer Vorstufe davon, nämlich einer Verbindung der allgemeinen Formel (III) 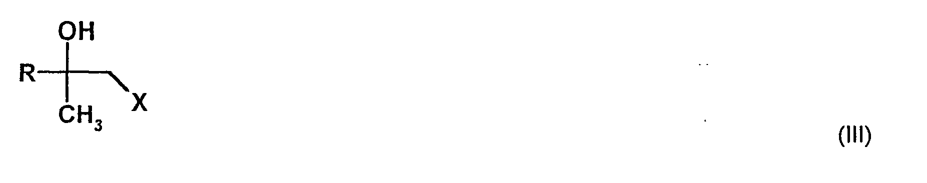 ausgegangen wird, kann die Abgangsgruppe X vom Fachmann entsprechend den gewählten Reaktionsbedingungen gewählt werden. Geeignete Abgangsgruppen sind dem Fachmann bekannt und umfassen Halogene, wie z.B. Cl, Br und 1, sowie reaktive Abgangsgruppen wie z.B. Alkali- und Arylsulfonate und insbesondere Mesylat, Tosylat und Brosylat.

Geeignete Edukte, in denen X ein Halogen ist, sind z.B. aus der WO 98/55153 bereits bekannt oder können aus der Epoxid-Vorstufe mittels konventioneller Verfahren der organischen Chemie einfach hergestellt werden.

Geeignete Edukte, in denen X eine gängige Abgangsgruppe wie z.B. eine Mesylat-, Brosylat- oder Tosylat-Gruppe ist, sind aus der WO 01/00608 bekannt und können aus der entsprechenden Alkoholvorstufe mittels konventioneller Verfahren der organischen Chemie erhalten werden.

Wenn in dem erfindungsgemäßen Verfahren an Stelle der Epoxidvorstufe unmittelbar das Epoxid eingesetzt werden soll, kann dieses z.B. wie in der EP 0 100 172 beschrieben erhalten werden.

Als p-Fluorphenylsulfinat-Salze werden erfindungsgemäß vorzugsweise Ammonium-p-fluorphenylsulfinat, Erdalkali-p-fluorphenylsulfinate oder Alkali-p-fluorphenylsulfinate und - besonders bevorzugt Natrium-p-fluorphenylsulfinat eingesetzt. Dieses kann auf bekanntem Weg, z.B. nach Olah, Pavlath, Acta Chim. Hung. 4 [1954] S. 111-117, in sehr guter Ausbeute erhalten werden.

Das erfindungsgemäße Verfahren weist darüber hinaus den Vorteil auf, daß die Öffnung des Epoxids das in dem Epoxid bereits vorhandene sterochemische Zentrum nicht beeinflußt, so daß das erfindungsgemäße Verfahren sowohl zur Herstellung des (±)-Bicalutamids als auch zur Herstellung des R-(-)- oder des S-(+)-Bicalutamids verwendet werden kann. Hierbei kommt es lediglich darauf an, ob das racemische Epoxid bzw. seine Halohydrin-Vorstufe oder ein entsprechendes optisch aktives R- bzw. S-Epoxid bzw. die entsprechende optisch aktive Halohydrin-Vorstufe als Edukt eingesetzt wird. Die erfindungsgemäße Umsetzung des Epoxids mit dem p-Fluorphenylsulfinat-Salz erfolgt vorzugsweise in einem geeigneten Lösungsmittel, wie z.B. Methanol oder DMF, in Gegenwart einer Säure, wie z.B. Eisessig, bei Raumtemperatur oder unter Erwärmen.

In einer bevorzugten Ausführungsform der Erfindung wird das Epoxid der allgemeinen Formel (1) in einem geeigneten Lösungsmittel wie Methanol nach der Zugabe von Eisessig unter Erwärmen auf ca. 50°C mit Natrium-p-fluorphenylsulfinat für ca. 5 h zur Reaktion gebracht. Nach Abschluß der Reaktion wird das Reaktionsgemisch nach Entfernen der flüchtigen Bestandteile durch ein wäßrig-organisches Extraktionsverfahren aufgearbeitet. Das nach dem Abziehen des organischen Lösungsmittels erhaltene Rohprodukt wird aus einem geeigneten Lösungsmittel, z.B. aus Diisopropylether, umkristallisiert, wobei reines Bicalutamid erhalten wird. Diese Reaktion wird durch das nachfolgende Reaktionsschema veranschaulicht:

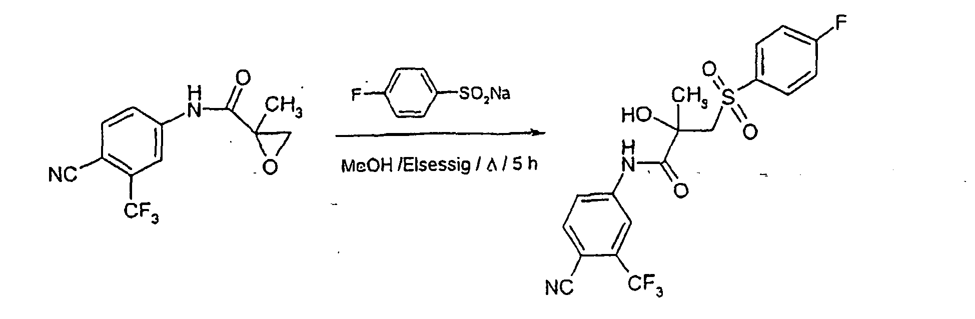

In einer weiteren bevorzugten Ausführungsform des erfindungsgemäßen Verfahrens wird zunächst aus einer offenkettigen Verbindung der allgemeinen Formel (III) in situ das entsprechende Oxiran erzeugt (z.B. durch Erwärmen der Verbindung der allgemeinen Formel (III) mit Kalium-tert-butylat in Toluol). In einem zweiten Schritt wird dann ohne Isolierung des gebildeten Oxirans dieses mit Natrium-p-fluorphenylsulfinat in einem geeigneten Lösungsmittel, wie z.B. Dimethylformamid (DMF), nach Zugabe von Eisessig bei Raumtemperatur oder leicht erhöhter Temperatur für ca. 16 h zur Reaktion gebracht. Diese Reaktion wird durch das nachfolgende Reaktionsschema veranschaulicht:

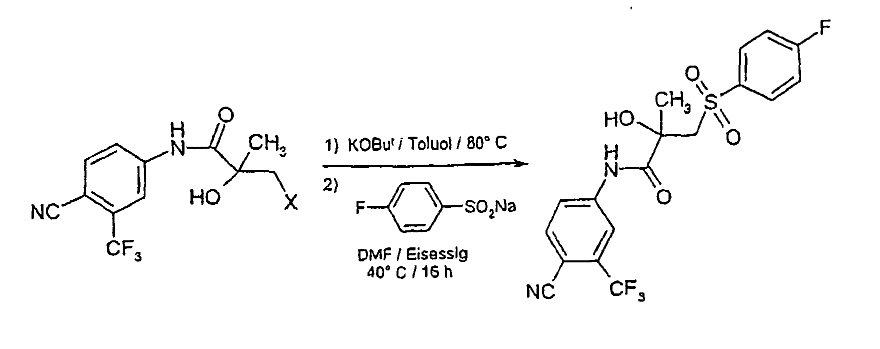

Die Ausbeute an Bicalutamid ist durch die Führung als Eintopfreaktion naturgemäß besser als bei einer Zwischenisolation des Oxirans, da z.B. ein normaler Aufarbeitungsverlust bei der Isolation des Oxirans vermieden wird. Zur Aufarbeitung kann der Reaktionsrückstand beispielsweise nach dem Abziehen flüchtiger Bestandteile in Dimethylformamid aufgenommen und mit Wasser verdünnt werden, wobei das Produkt auskristallisiert. Falls erforderlich, kann sich eine weitere Reinigung durch Umkristallisation aus einem geeigneten Lösungsmittel, wie z.B. Diisopropylether, anschließen.

Das erfindungsgemäße Verfahren wird durch die folgenden Beispiele näher erläutert:

### Beispiel 1

### Synthese von Bicalutamid aus der Epoxid-Vorstufe

In einem Vierhalskolben werden 7,0 g (25,9 mmol) N-(4'-Cyano-3'-trifluormethylphenyl)-2-methyl-oxiran-2-carboxamid, 9,43 g (51,8 mmol) Natrium-p-fluorphenylsulfinat in 50 ml Methanol und 3 ml Eisessig vorgelegt und anschließend 5 Stunden unter Rückfluß erhitzt.

Nach Abschluß der Reaktion wird das Reaktionsgemisch zur Trockne evaporiert und der Rückstand wird mit 50 ml CH₂Cl₂ und 20 ml Wasser aufgenommen. Die organische Phase wird mehrmals mit Wasser gewaschen und über MgSO₄ getrocknet. Nach dem Abziehen des Lösungsmittels erhält man rohes Bicalutamid, das zur weiteren Reinigung aus Diisopropylether umkristallisiert wird.

| | |
|---|---|
| Ausbeute: | 7,1 g |
| Schmelzpunkt: | 187° - 189°C |
| Reinheit: | 96,6 % (HPLC) |

### Beispiel 2

### Synthese des Bicalutamids aus der Halohydrin-Vorstufe (Eintopf-Variante)

3,07 g 3-Chlor-N-(4'-cyano-3-trifluormethylphenyl)-2-hydroxypropionamid werden in 50 ml Toluol suspendiert und auf 80°C erwärmt. Anschließend werden unter intensivem Rühren über 30 min 1,12 g Kalium-t-butylat in 20 ml Toluol zugegeben. Danach wird bei gleicher Temperatur noch 30 min nachgerührt.

Nach dem Abkühlen auf Raumtemperatur werden 3,64 g Natrium-p-fluorphenylsulfinat, 0,6 ml Eisessig und 50 ml DMF zugegeben; die erhaltene Suspension wird anschließend 16 h bei 40°C gerührt. Nach dem Entfernen der flüchtigen Bestandteile im Vakuum wird der Rückstand in wenig DMF aufgelöst und anschließend mit Wasser verdünnt. Dabei fällt Bicalutamid aus der Lösung aus; das Produkt wird abfiltriert, mit Wasser gewaschen und im Vakuum getrocknet.

| | |
|---|---|
| Ausbeute: | 3,9 g |
| Schmelzpunkt: | 187° - 189°C |
| Reinheit: | 95,8 % (HPLC) |

## Patentansprüche

1. Verfahren zur Herstellung von N-(4'-Cyano-3'-trifluormethyl)-3-(4"-fluorphenylsulfonyl)-2-hydroxy-2-methyl-propionamid (Bicalutamid), **dadurch gekennzeichnet, daß** ein Epoxid der allgemeinen Formel (I) 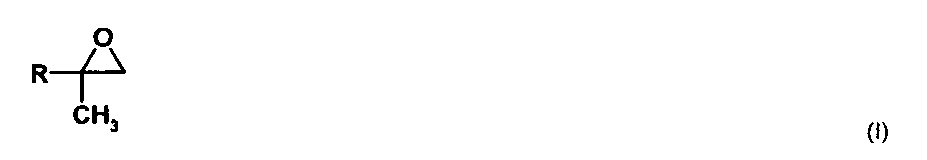 worin R ein Rest der Formel (II) 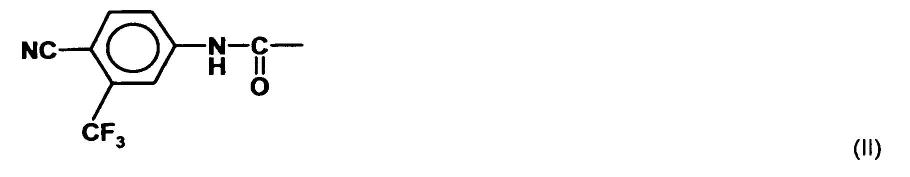 oder eine Vorstufe davon ist, mit einem p-Fluorphenylsulfinat-Salz in Gegenwart einer Säure umgesetzt wird und, wenn R eine Vorstufe des Restes der Formel (11) ist, diese in einen Rest der Formel (II) überführt wird, wobei die Vorstufe aus Carboxyl, Säurehalogeniden, normalen Estern und aktivierten Estern ausgewählt ist.

2. Verfahren zur Herstellung von N-(4'-Cyano-3'-trifluormethyl)-3-(4"-fluorphenylsulfonyl)-2-hydroxy-2-methyl-propionamid (Bicalutamid), **dadurch gekennzeichnet, daß** eine Verbindung der allgemeinen Formel (III) 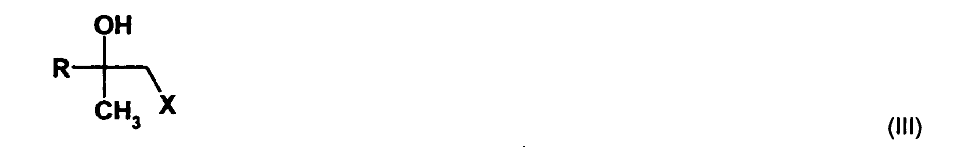 worin R ein Rest der Formel (II) 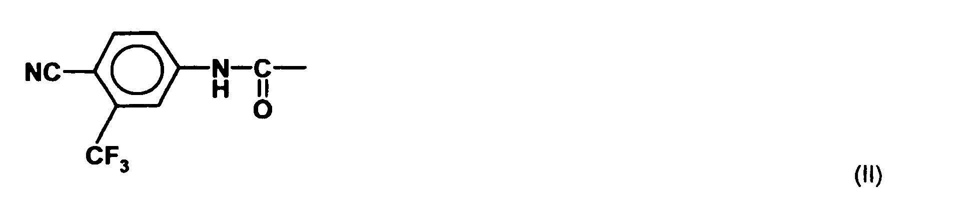 oder eine Vorstufe davon ist und X eine Abgangsgruppe ist, mit einem p-Fluorphenylsulfinat-Salz in Gegenwart einer Säure umgesetzt wird und, wenn R eine Vorstufe des Restes der Formel (II) ist, diese in einen Rest der Formel (II) überführt wird, wobei die Vorstufe aus Carboxyl, Säurehalogeniden, normalen Estern und aktivierten Estern ausgewählt ist.

3. Verfahren nach Anspruch 1, **dadurch gekennzeichnet, daß** das Epoxid der allgemeinen Formel (I) aus einer Verbindung der allgemeinen Formel (III) 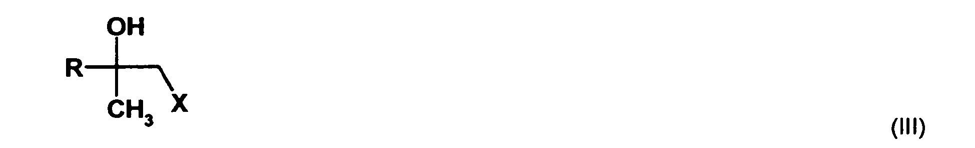 worin R wie in Anspruch 1 definiert ist und X eine Abgangsgruppe ist, erhalten wird.

4. Verfahren nach Anspruch 3, **dadurch gekennzeichnet, daß** die Umsetzung ausgehend von der Verbindung der allgemeinen Formel (III) bis zur Reaktion mit dem p-Fluorphenylsulfinat-Salz in einer Eintopfreaktion durchgeführt wird.

5. Verfahren nach einem der Ansprüche 2-4, **dadurch gekennzeichnet, daß** X ausgewählt ist aus der Gruppe bestehend aus Halogenen, wie Cl, Br und 1, und Alkyl- und Arylsulfonaten wie Mesylat, Tosylat und Brosylat.

6. Verfahren nach einem der vorhergehenden Ansprüche, **dadurch gekennzeichnet, daß** das Epoxid der allgemeinen Formel (I) oder die Verbindung der allgemeinen Formel (III) in Form ihres Racemates oder eines ihrer optisch aktiven R- bzw. S-Enantiomeren eingesetzt wird.

7. Verfahren nach einem der vorhergehenden Ansprüche, **dadurch gekennzeichnet, daß** das p-Fluorphenylsulfinat-Salz als Alkali-p-fluorphenylsulfinat und insbesondere als Natrium-p-fluorphenylsulfinat eingesetzt wird.

## Claims

1. A process for preparing N-(4'-cyano-3'-trifluoromethyl)-3-(4"-fluorophenylsulfonyl)-2-hydroxy-2-methylpropionamide (bicalutamide), wherein an epoxide of the general formula (1) 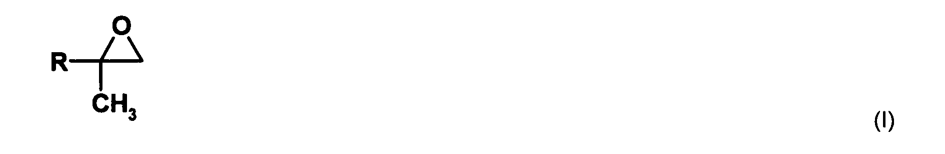 in which R is a radical of the formula (II) 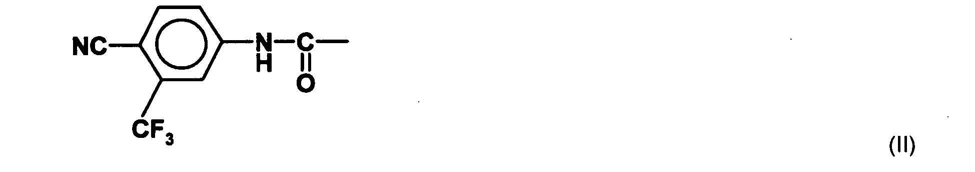 or a precursor thereof is reacted with a p-fluorophenylsulfinate salt in the presence of an acid and, if R is a precursor of the radical of the formula (II), the latter is converted into a radical of the formula (II), wherein the precursor is selected from the group consisting of carboxyl, acid halides, normal esters and activated esters.

2. A process for preparing N-(4'-cyano-3'-trifluoromethyl)-3-(4"-fluorophenylsulfonyl)-2-hydroxy-2-methylpropionamide (bicalutamide), wherein a compound of the general formula (III) 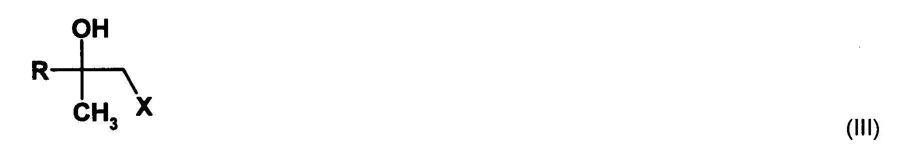 in which R is a radical of the formula (II) 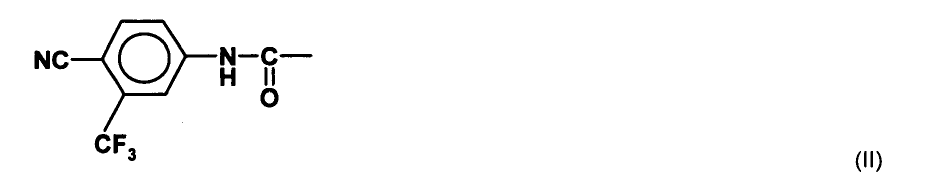 or a precursor thereof, and X is a leaving group, is reacted with a p-fluorophenylsulfinate salt in the presence of an acid and, if R is a precursor of the radical of the formula (II), the latter is converted into a radical of the formula (II), wherein the precursor is selected from the group consisting of carboxyl, acid halides, normal esters and activated esters.

3. The process as claimed in claim 1, in which the epoxide of the general formula (I) is obtained from a compound of the general formula (III) 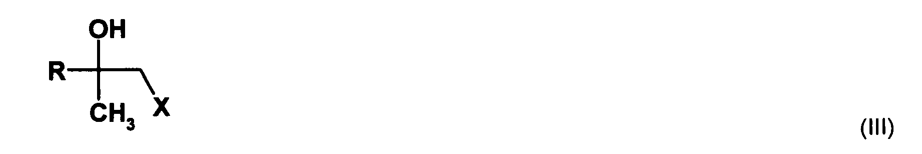 in which R is as defined in claim 1, and X is a leaving group.

4. The process as claimed in claim 3, in which the reaction is carried out starting from the compound of the general formula (III) up to the reaction with the p-fluorophenylsulfinate salt in a one-pot reaction.

5. The process as claimed in any of claims 2-4, in which X is selected from the group consisting of halogens such as Cl, Br and I, and alkyl- and arylsulfonates such as mesylate, tosylate and brosylate.

6. The process as claimed in any of the preceding claims, in which the epoxide of the general formula (I) or the compound of the general formula (III) is employed in the form of its racemate or of one of its optically active R or S enantiomers.

7. The process as claimed in any of the preceding claims, in which the p-fluorophenylsulfinate salt is employed as alkali metal p-fluorophenylsulfinate and in particular as sodium p-fluorophenylsulfinate.

## Revendications

1. Procédé pour la préparation de N-(4'-cyano-3'-trifluorométhyl)-3-(4"-fluorophénylsulfonyl)-2-hydroxy-2-méthyl-propionamide (bicalutamide), **caractérisé en ce qu'**un époxyde de formule générale (I) 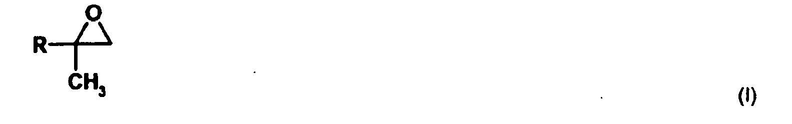 dans laquelle R est un radical de formule (II) 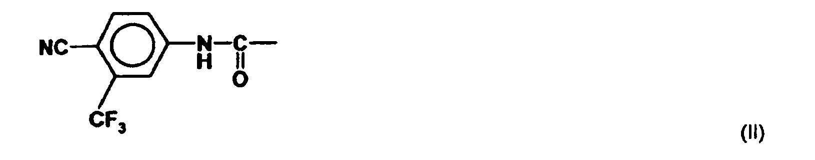 ou un précurseur de celui-ci, est amené à réagir avec un sel de p-fluorophénylsulfinate en présence d'un acide et, quand R est un précurseur du radical de formule (II), celui-ci est converti en un radical de formule (II), le précurseur étant sélectionné parmi du carboxyle, des halogénures d'acide, des esters normaux et des esters activés.

2. Procédé pour la préparation de N-(4'-cyano-3'-trifluorométhyl)-3-(4"-fluorophénylsulfonyl)-2-hydroxy-2-méthyl-propionamide (bicalutamide), **caractérisé en ce qu'**un composé de formule générale (III) 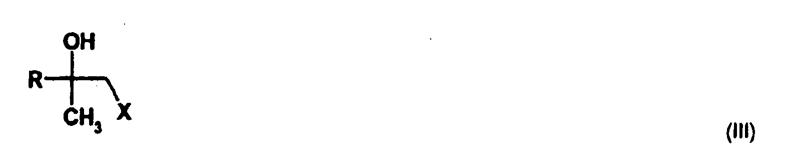 dans laquelle R est un radical de formule (II) 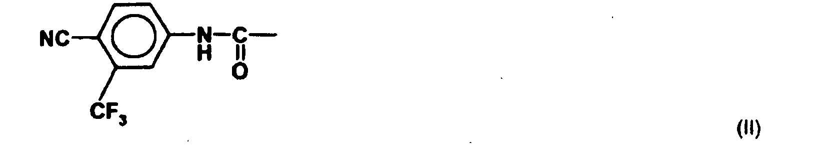 ou un précurseur de celui-ci et X est un groupe de départ, est amené à réagir avec un sel de p-fluorophénylsulfinate en présence d'un acide et, quand R est un précurseur du radical de formule (II), celui-ci est converti en un radical de formule (II), le précurseur étant sélectionné parmi du carboxyle, des halogénures d'acide, des esters normaux et des esters activés.

3. Procédé selon la revendication 1, **caractérisé en ce que** l'époxyde de formule générale (I) est obtenu à partir d'un composé de formule générale (III) 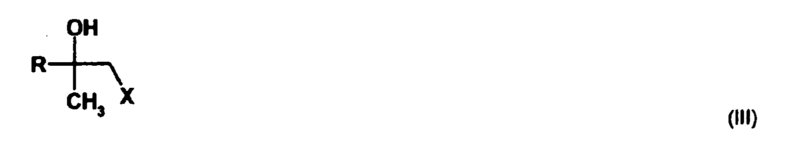 dans laquelle R est comme défini dans la revendication 1 et X est un groupe de départ.

4. Procédé selon la revendication 3, **caractérisé en ce que** la réaction à partir du composé de formule générale (III) et allant est mise en oeuvre dans une réaction à un composant jusqu'à la réaction avec le sel de p-fluorophénylsulfinate.

5. Procédé selon l'une quelconque des revendications 2 à 4, **caractérisé en ce que** X est sélectionné parmi le groupe constitué des halogènes, comme Cl, Br et I, et des alkylsulfonates et arylsulfonates comme le mésylate, le tosylate et le brosylate.

6. Procédé selon l'une quelconque des revendications précédentes, **caractérisé en ce que** l'époxyde de formule générale (I) ou le composé de formule générale (III) est utilisé sous la forme de son racémique ou d'un de ses énantiomères R ou S optiquement actifs.

7. Procédé selon l'une quelconque des revendications précédentes, **caractérisé en ce que** le sel de p-fluorophénylsulfinate est utilisé en tant que p-fluorophénylsulfinate alcalin et en particulier en tant que p-fluorophénylsulfinate de sodium.
